# EUROPEAN PATENT SPECIFICATION

(11) **EP 0 392 601 B1**
(45) Date of publication and mention of the grant of the patent: **20.07.1994**
(21) Application number: 90200829.1
(22) Date of filing: 05.04.1990
(51) Int. Cl.: C07C 67/38

(54) **Process for the preparation of an alkyl methacrylate**
Verfahren zur Herstellung eines Alkylmethacrylates
Procédé de préparation d'un méthacrylate d'alkyle

(30) Priority: 11.04.1989 GB 8908079
(43) Date of publication of application: 17.10.1990
(73) Proprietor: SHELL INTERNATIONALE RESEARCH MAATSCHAPPIJ B.V., 2596 HR Den Haag (NL)
(72) Inventor: Doyle, Michael John, NL-1031 CM Amsterdam (NL); Van Gogh, Johan, NL-1031 CM Amsterdam (NL); Van Ravenswaay Claasen, Johan Christiaan, NL-1031 CM Amsterdam (NL)

(56) References cited:
- EP-A- 0 271 144
- DE-A- 1 952 976
- GB-A- 997 923
- BULLETIN OF THE CHEMICAL SOCIETY OF JAPAN. vol. 37, no. 11, November 1964,TOKYO JP pages 1601 - 1609; Y. SAKAKIBARA: 'THE SYNTHESIS OF METHACRYLIC ESTERS BY CARBOXYLATION REACTION OF METHYLACETYLENE.I.METHYL METHACRYLATE; REACTION,CONDITIONS AND PRODUCTS'

## Description

The present invention relates to a process for the preparation of an alkyl methacrylate, especially methyl methacrylate.

Methyl methacrylate is prepared in industry mainly by the so-called acetone cyanohydrin process. This process presents disadvantages in that large quantities of waste sulphuric acid and ammonium bisulphate are produced, which have to be discharged or worked up for reuse, and in that another waste material, HCN, is highly toxic and pressures against its storage and transportation are increasing (cf. T. Haeberle and G. Emig in Chem. Eng. Technol. 11,6 (1988) 392-402). As concerns about the environment have increased, considerable research has been devoted to finding alternative processes which do not present these disadvantages.

One possible alternative process, described in 1964 by Y. Sakakibira in Bull. Chem. Soc. Japan 37, 11 (1964) 1601-1609, comprises the reaction of propyne with carbon monoxide and an alkanol in the presence of a carboxylation catalyst. Although this process has now been known for a long time, and has attracted a considerable amount of interest, it has never been commercialized.

A factor inhibiting the commercial exploitation of the carboxylation process has been the unavailability of large quantities of a suitable low-priced propyne feed.

Many processes have been described for the preparation of propyne. For example, the chapter "Methylacetylene" in Kirk-Othmer's Encyclopaedia of Chemical Technology, 2nd ed., Volume Supplement (1971), pages 547 to 556 refers to various processes including the dehydrohalogenation of propylene dibromide, the hydration of magnesium carbide, the reaction of sodium acetylide and dimethylsulphate in liquid ammonia, and a variety of pyrolysis or cracking methods.

Of the many potential sources of a propyne feed for the preparation of alkyl methacrylates, the cheapest appears to be the C₃-stream produced by an ethene cracker (also known as a naphtha cracker, a gas oil cracker and/or an LPG cracker), a catalytic cracker or an LPG (liquefied petroleum gas)-dehydrogenation process. A characteristic of such a stream is that it comprises a mixture of propyne and propadiene in an approximate ratio of 1 to 1. At present, the mixture of propyne and propadiene is generally not separated, but is usually burnt (either as a flare or as welding gas) or is hydrogenated to propene and propane.

European patent application publication number EP-A-0190473 discloses a process for the preparation of alkyl acrylates, such as methyl methacrylate, by the carboxylation of propadiene. Example 10 in the specification describes an experiment in which methyl methacrylate is prepared by reacting a mixture of propadiene and propyne with carbon monoxide and methanol in the presence of a relatively inactive carboxylation catalyst. Both the propadiene and the propyne are converted into methyl methacrylate. The ratio of propyne to propadiene is 1 to 1, the same as the ratio found in a C₃-stream produced by an ethene cracker, a catalytic cracker or an LPG-dehydrogenation process.

Surprisingly, it has now been found that propadiene poisons carboxylation catalysts in the carboxylation of propyne and methanol to give methyl methacrylate. Moreover, the poisoning effect of propadiene appears to increase as the intrinsic activity of the carboxylation catalyst for propyne carboxylation increases.

Accordingly, the invention provides a process for the preparation of an alkyl methacrylate, which comprises: a) selectively removing propadiene from a C₃-mixture comprising a mixture of propyne and propadiene that has been obtained from an ethene cracker, a catalytic cracker or an LPG-dehydrogenation process, to afford a propyne feed in which the ratio of propyne to propadiene is ≧ 6, and b) contacting the propyne feed with carbon monoxide and an alkanol in the presence of a carboxylation catalyst.

The C₃-mixture used as the starting material in the process according to the invention comprises a mixture of propyne and propadiene that has been obtained from an ethene cracker, a catalytic cracker or an LPG-dehydrogenation process. Ethene crackers, catalytic crackers and LPG-dehydrogenation processes are well known in the art.

Thus an ethene cracker is a type of cracker in which ethene is prepared from hydrocarbon fractions such as naphtha, gas oil, LPG (preferably isobutane) or ethane by thermal cracking. A catalytic cracker is a cracker in which hydrocarbons are prepared by the catalytic cracking of hydrocarbon fractions such as heavy gas oil or vacuum distillates. An LPG-dehydrogenation process is a process in which propane is converted into propene, either thermally or catalytically. Each of these processes provides, amongst others, a C₃-stream consisting mainly of C₃-hydrocarbons, in particular propane, propene, propyne and propadiene.

Preferably the C₃-mixture comprises a mixture of propyne and propadiene that has been obtained from an ethene cracker.

The C₃-mixture used in the process according to the invention may be a C₃-stream produced by an ethene cracker, a catalytic cracker or an LPG-dehydrogenation process. However, it is preferably a mixture derived from such a C₃-stream by a process in which the concentration of the mixture of propyne and propadiene has been increased. For example, it may be a mixture derived from such a C₃-stream by distilling-off propane and/or propene, by selective scrubbing with a solvent and/or by adding more propyne and/or propadiene. More propyne and/or propadiene may be added, for example by way of a recycle to be described in greater detail hereinafter.

In some cases, it will be very convenient to combine mixtures of propyne and propadiene obtained from several different ethene crackers, catalytic crackers and/or LPG-dehydrogenation processes, which may be located at different refineries. Such mixtures will most conveniently be transported in concentrated form.

An attractive way of obtaining the C₃-mixture from an ethene cracker plant comprises taking at least part of the "crude" C₃-stream obtained from an already present depropaniser (a distillation column where C₃-hydrocarbons are separated from higher hydrocarbons, bypassing the hydrogenator (where normally the propyne, propadiene and some propene are hydrogenated to a propane/propene mixture, whence the latter mixture is fed to a propane/propene splitter from which "polymer grade" propene is obtained as a top stream and essentially pure propane as a bottom stream) and introducing it directly into a or the propane/propene splitter. In this embodiment, it is advantageous to operate the said splitter under slightly different conditions than when operating with hydrogenation as described above, to account for the fact that at least part of its feed still contains some propyne and/or propadiene, as it has not been subjected to a preliminary hydrogenation. It would then be operated thus, that the top stream of the splitter would still contain "polymer grade" propene, though in lesser yield, and some of the propene would leave the splitter at the bottom with a stream which consists of mainly propane and propene (about 70%) and further comprises propadiene and propyne in roughly equimolar portions.

It is advantageous to minimise the amount of inert or quasi-inert materials in the propyne feed in order to maximise the throughput of a plant having a fixed capacity at a given catalyst activity. Thus, according to a preferred aspect of the invention, the mixture of propyne and propadiene in the C₃-mixture has been concentrated by selective scrubbing with a solvent, whereby a solvent stream containing the C₃-mixture is obtained. For example, the mixture of propyne and propadiene may have been obtained from the bottom effluent of a propane/propene splitter, which contains significant amounts of propane, propene, propyne and propadiene. The scrubbing is suitably carried out in a column under elevated pressure (2-20, preferably 6-12 bar) using countercurrent flows of an organic solvent and the bottom effluent of the propane/propene splitter, so that typically a stream consisting essentially of propane and propene (and < 0.2 % of propyne/propadiene) is removed as the overhead fraction.

The solvent which absorbs propyne and at the elevated pressure employed, also propadiene, suitably comprises a polar organic solvent, such as an amide, e.g. dimethylformamide, dimethylacetamide or N-methylpyrrolidone, a nitrile such as acetonitrile, a sulphone such as sulpholane, or a mixture thereof, particularly dimethylformamide. Another preferred extraction solvent is an alcohol such as methanol, which presents the advantage that it is used too as one of the reactants in the subsequent carboxylation reaction, and thus simplifies the management of the process.

The propadiene may be selectively removed by chemical means, such as by isomerization to propyne, and/or by physical means such as by distillation, preferably extractive distillation.

The isomerization of propadiene into propyne is a chemical equilibrium reaction and is well known in the art. The position of chemical equilibrium depends upon the temperature. Thus as the temperature is increased, the proportion of propadiene increases. At ambient temperature, the ratio of propyne to propadiene obtained by isomerization is approximately 9 to 1.

The isomerization is conveniently effected in the gas or liquid phase in the presence of an isomerization catalyst at a temperature in the range of from -30 to 100 °C, preferably 0 to 40 °C, more preferably 10 to 30 °C, and at a pressure in the range of from 0.1 to 100 bar, more preferably 1 to 20 bar.

Catalysts suitable for isomerizing propadiene into propyne are well known in the art. For example, the isomerization catalyst may comprise an alkali metal or alkali metal oxide deposited on alumina, such as a composition obtainable by heating an alkali metal carbonate deposited on an alumina carrier, preferably K₂CO₃ on gamma alumina, in an inert atmosphere, or a composition obtainable by depositing at least one molten alkali metal on alumina, preferably the low melting eutectic mixture of potassium and sodium on alumina. Suitable isomerization catalysts are also described in Kirk-Othmer's Encyclopaedia of Chemical Technology, 2nd ed., Volume Supplement (1971), pages 547 to 556, and in US patent No. 3,671,605.

In general, the activity of isomerization catalysts decreases with decreasing temperature. Accordingly, when it is desired to prepare a propyne feed in which the ratio of propyne to propadiene is ≧ 10, especially ≧ 20, it is preferable to remove propadiene from the C₃-mixture by physical separation means.

When propadiene has been removed by physical separation means it may then be reacted with carbon monoxide and an alkanol to afford alkyl methacrylate. However, it is preferably isomerized to a mixture of propyne and propadiene, and recycled to step a) or step b) of the process. Optionally, the product of the isomerization is subjected to a distillation to remove heavy ends prior to recycling to step a) or step b).

Extractive distillation is a method well known for removing one component from a mixture comprising two very similar components. Thus, for the removal of propadiene from a mixture of propyne and propadiene, the mixture of propyne and propadiene is dissolved in a polar organic solvent, and propadiene is removed as a gas (e.g. by stripping) leaving propyne dissolved in the solvent. Suitable solvents include amides, for example dimethyl formamide or N-methylpyrrolidone, nitriles such as acetonitrile, sulphones such as sulpholane and alcohols such as methanol. Dimethylformamide, N-methylpyrrolidone, methanol and mixtures thereof are preferred solvents.

It will be appreciated that by combining a physical separation step for the removal of propadiene with an isomerization step, all of the propyne and propadiene in the original C₃-mixture can in principle be converted into alkyl methacrylate. This combination of process steps therefore constitutes a particularly preferred aspect of the invention.

When the C₃-mixture has been obtained by scrubbing with a solvent, propadiene is advantageously removed by stripping from the propyne and propadiene containing solvent stream to afford the propyne feed. This stripping is possible because propadiene and propyne exhibit different volatilities and solubilities in solvents. The stripping may suitably take place in a column downstream of the main absorption column, employing indirect heat exchange in the bottom of the column. The propyne may then, if desired, be separated from the solvent in a further column. The stripping operation can be adjusted to a propadiene content in the propyne feed of almost zero.

In the process according to the invention, the molar concentration of propyne in the propyne feed preferably lies above 35%, more preferably above 50%, even more preferably above 90%, most preferably at least 99%. Thus the feed mixture preferably contains at most 10% (molar) of propadiene and at least 35% (molar) of propyne.

The propyne feed preferably comprises at least 50% (mass) of propyne and propadiene combined, more preferably at least 60%, even more preferably at least 80%.

The propyne feed preferably comprises at least 50%, especially at least 90% (molar), of C₃-hydrocarbons.

In the process according to the invention, it is preferred to use a propyne feed in which the ratio of propyne to propadiene is at least 8, particularly ≧ 20, and especially ≧ 100. With highly active carboxylation catalysts, it is preferable to use a propyne feed in which the ratio is ≧ 500, more preferably ≧ 1000, most preferably ≧ 10,000.

The carboxylation catalyst used in the process according to the invention may be any catalyst having activity for the carboxylation of propyne. It is preferably a Group VIII metal catalyst, more preferably a palladium catalyst.

Preferably the carboxylation catalyst is based on a composition of a Group VIII (e.g. palladium) compound, a ligand (e.g. a monodentate or quasi-bidentate phosphine, arsine, stibine or a similar nitrogen compound) and an anion of a Broensted acid (from a salt, ester, anhydride or acid, and preferably not too strongly coordinating). A particularly preferred example of such a catalyst is based on a composition of a palladium(II) compound, an organic phosphine of formula PR₃ in which each R independently stands for an optionally substituted hydrocarbyl or heterocyclic group, and a non-hydrohalogenic Broensted acid having a pK_{A} < 2.

A hydrocarbyl group in an optionally substituted hydrocarbyl group is preferably an alkyl group, for example a C₁₋₆ alkyl group such as methyl, ethyl, propyl, isopropyl, butyl, isobutyl, or t-butyl, a cycloalkyl group, e.g. cyclopentyl or cyclohexyl, or an aryl group such as phenyl or naphthyl. Two R-groups may alternatively represent an optionally substituted alkylene chain.

A heterocyclic group in an optionally substituted heterocyclic group is preferably an aromatic group having an imino nitrogen, for example a pyridyl, pyrazinyl, quinolyl, isoquinolyl, pyrimidinyl, pyridazinyl, cinnolinyl, triazinyl, quinoxalinyl or quinazolinyl group. An imino nitrogen atom in an aromatic group having an imino nitrogen atom is preferably connected to phosphorus through a single bridging nitrogen atom, as for example in 2-pyridyl, 2-pyrazinyl, 2-quinolyl, 1-isoquinolyl, 3-isoquinolyl, 2-pyrimidinyl, 3-pyridazinyl, 3-cinnolinyl, 2-triazinyl, 2-quinoxalinyl and 2-quinazolinyl.

Examples of optional substituents which may be present in an optionally substituted hydrocarbyl or heterocyclic group include halogen atoms, e.g. fluorine, chlorine or bromine; alkyl groups, e.g. methyl or ethyl; haloalkyl groups, e.g. trifluoromethyl; alkoxy groups, e.g. methoxy or ethoxy; haloalkoxy groups, e.g. trifluoromethoxy; acyl groups, e.g. acetyl; acyloxy groups, e.g. acetoxy; amino groups, e.g. dimethylamino; hydroxyl groups; nitrile groups; acylamino groups, e.g. acetamido; and aryl groups, e.g. phenyl.

A non-halogenic Broensted acid may be, for example, sulphuric acid, a sulphonic acid such as p-toluenesulphonic acid, naphthalenesulphonic acid, trifluoromethanesulphonic acid, chlorosulphonic acid, fluorosulphonic acid or a sulphonated ion exchange resin; a phosphoric acid such as orthophosphoric acid, pyrophosphoric acid or benzene phosphoric acid; a carboxylic acid such as trifluoroacetic acid; a perhalic acid such as perchloric acid; fluorosilicic acid; HBF₄; HPF₆ or HSbF₆.

Examples of such catalysts are mentioned in Applicant's EP-A-186228 and Ep-A-271144, e.g. combinations of (a) palladium acetate, (b) triphenylphosphine, tris(p-methoxyphenyl)phosphine, or diphenyl-2-pyridylphosphine, and (c) p-toluenesulphonic or trifluoroacetic acid.

The reaction between propyne, the alcohol and carbon monoxide is preferably effected at a temperature in the range of from 20 to 200 °C, more preferably 20 to 80 °C, and at a pressure in the range of from 5 to 70 bar. A separate solvent is not essential for the reaction. However, an ester of the alcohol may conveniently be used as solvent.

When the carboxylation catalyst is a Group VIII metal catalyst, it is preferred that the catalyst has a conversion activity in the absence of propadiene of at least 100 g propyne/g of catalytic metal/hour, more preferably 1,000 g propyne/g of catalytic metal/hour, preferably of at least 5,000, more preferably of at least 10,000 g propyne/g of catalytic metal/hour. This is equivalent roughly to a production of 25 kg methacrylate/g catalytic metal/hour, where the catalytic metal is palladium and the methacrylate is methyl methacrylate.

The alkyl methacrylate which is the product of the present process, is suitably an ester of an alcohol having up to 20, preferably 1 to 4, carbon atoms. Examples of alcohols are methanol, ethanol, propanol, iso-propanol, butanol, iso-butanol and tert-butanol. Most preferably the alkanol is methanol, thus giving methyl methacrylate as the product.

A suitable and attractive method for carrying out the carboxylation reaction involves that the propyne feed is combined with a mixture of fresh alkanol and a recycle stream of an methacrylate/alkanol azeotrope, and then fed into the reactor simultaneously with (a solution of) the catalyst and carbon monoxide.

This is further described using methanol as the preferred alkanol feed. Very suitably the propyne feed is brought into a mixing device, e.g. a tank, to combine it with a mixture of fresh methanol and a recycle stream of a methyl methacrylate/methanol azeotrope, and then fed into the reactor, and concomitant with that, the catalyst solution is also fed into the reactor. One could introduce the catalyst solution using the CO feed pipe or separately. The reactor effluent product stream is flashed isothermally and stripped of unreacted gases. This gas stream may be chilled (to about -20 °C) to recondense valuable low volatile components which are returned to the liquid feed. The uncondensed gas (mainly CO) is removed and used elsewhere, e.g. as a fuel. Any unreacted propyne present herein can be scrubbed out with methanol and recycled to the reactor. The liquid fraction which contains product, catalyst residues and heavy ends, is fed into a distillation column.

The column top stream which consists of an azeotrope of methyl methacrylate and methanol, is recycled to the feed mixing device and subsequently to the reactor. The bottom stream is suitably fed to a second distillation column. The top product from this column is pure methyl methacrylate, whereas the bottom product contains some methacrylate, catalyst residues and heavy ends. The latter methyl methacrylate may be recovered in a heavy ends stripper and returned to the second column. The concentrated residue may be worked up for reuse or for disposal in a responsible way.

The invention will now be illustrated in more detail by the following Examples. Example 1 illustrates a process in which a C₃-mixture typical of that of a C₃-steam from a cracker is converted into a propyne feed by scrubbing with a solvent followed by stripping off of propadiene. Example 2 illustrates a process in which propadiene is isomerized over an isomerization catalyst to afford propyne. Examples 3 and 4 illustrate the carboxylation of propyne to afford methyl methacrylate. The Comparative Examples demonstrate the surprising poisoning effect of propadiene on the carbonylation of propyne.

### EXAMPLE 1

A fresh feed, consisting of 52.7 mole % propane, 1.6 mole % of propylene, 19.1 mole % of propadiene and 25.1 mole % of propyne and 1.5 % of heavier hydrocarbons, is combined with a recycle stream from a propadiene isomerization reactor and fed to an absorbtion column. If necessary the feed is first sent to a distillation column to remove high boiling impurities. The combined mixture contains 45.4% propane, 10.5% propylene, 11.4% propadiene and 32.7% propyne.

In the absorber the combined mixture is contacted with DMF (dimethylformamide) (2.8 kg DMF/kg combined mixture). The absorber is operated at 8 bar and is provided with a reboiler and a condensor. The liquid top product consists of 96.8% of propane, 3.0% propylene, 0.2% of propadiene and propyne combined. The bottom product consists of 68.3% DMF, 4.8% propadiene and 14.2% propyne, 8.6% propane and 4.1% propylene.

This mixture is cooled to 35 °C and fed to a first reboiled stripper provided with a condensor, operating at 2.6 bar, where all the propane, propylene, propadiene and part of the propyne are stripped off, resulting in a bottom product consisting of 88.1% DMF and 11.9% methylacetylene with only 10 ppm propadiene. The top product, consisting of 38.2% propane, 18.3% propylene, 21.5% propadiene and 22% propyne is fed to the propadiene isomerization reactor containing an isomerization catalyst where part of the propadiene is converted to propyne, resulting in an effluent containing 4.4% propadiene and 39.1% propyne. This effluent is the recycle stream which is combined with the fresh feed.

The bottom product is fed to a second reboiled stripper equipped with a condensor operating at 1.6 bar. The top product of this stripper consists of propyne containing only 80 ppm propadiene. The bottom product consists of DMF containing 0.5% propyne, which, after being cooled, is used again in the first absorber. The purpose of the condensors in the strippers is not only to condense the hydrocarbon vapours, but also to remove DMF from the hydrocarbon product.

### EXAMPLE 2

### Preparation of catalyst

A 20 %w potassium carbonate on alumina catalyst was prepared as follows:
- 250 g of 1/16˝ cylindrical gamma-Al₂O₃ (gamma-alumina) extrudates (pore volume = 0.7 ml/g) were activated during 16 hours at 500 °C.
- 50 g of K₂CO₃ (potassium carbonate, Baker analyzed) was dissolved in 150 ml of demineralized water at ambient temperature.
- 200 g of the activated gamma-Al₂O₃ was contacted with the above K₂CO₃ solution and well mixed (incipient wetness method).

After impregnation the catalyst was dried at 125-140 °C for 16 h. Prior to use the thus obtained 20 %w K₂CO₃ on gamma-Al₂O₃ was activated under nitrogen at 575 °C during 24 hour.

### Isomerization of propadiene

Propadiene was isomerized to propyne in a packed bed reactor containing activated K₂CO₃ on gamma-Al₂O₃ catalyst according to the following procedure:
- A 0.9 cm i.d. stainless steel tube reactor was filled with 2.0 g of K₂CO₃ on gamma-Al₂O₃ catalyst particles.
- The catalyst was activated according to the temperature treatment described above.

The reactor was fitted in an experimental set-up and a feed mixture of liquefied C₃- and C₄-hydrocarbons was pumped over the catalyst. The feed contained 15 %v propadiene, 22 %v propyne, 49 %v propene, 5 %v propane, 3 %v 1,3-butadiene and some minor amounts of other C₃-C₄-hydrocarbons.

At liquid hourly space velocities up to 10 l (feed)/l(reactor).hr propyne/propadiene isomerization equilibrium was established. The propyne and propadiene concentrations in the reaction product from the above described feed amounted 33.7 %v and 3.3 %v, when the reaction was carried out at 25 °C.

### EXAMPLE 3

A 250 ml magnetically stirred autoclave was filled with 0.1 mmol palladium acetate, 1 mmol tri(p-trifluoromethylphenyl)phosphine, 1 mmol methanesulphonic acid, 10 ml methanol and 40 ml anisole.

Air was then evacuated from the autoclave, and then 30 ml propyne was added. Then carbon monoxide was added to a pressure of 20 bar. The autoclave was then sealed and heated to 90 °C. Upon completion of the reaction, the contents of the autoclave were analyzed by gas liquid chromatography. The reaction rate was calculated to be 40 g propyne/g Pd/hour.

### Comparative Example A

The method of Example 3 was repeated, but using 15 ml propyne and 15 ml allene. The reaction rate was calculated to be 4 g propyne and allene/g Pd/hour.

### EXAMPLE 4

A 300 ml magnetically stirred stainless steel autoclave was successively filled with 0.025 mmol palladium(II) acetate, 1 mmol bis(6-methyl-2-pyridyl)phenylphosphine, 2 mmol paratoluenesulphonic acid, 30 ml N-methylpyrrolidone and 30 ml methanol. Air was evacuated from the autoclave, whereupon 25 ml propyne was added. Subsequently, carbon monoxide was added to a pressure of 60 bar. The autoclave was sealed and heated to a temperature of 80 °C. After a reaction time of 1.5 hours at 80 °C a specimen of the contents was analysed by means of gas liquid chromatography. The mean conversion rate was calculated to be 7500 g propyne/g Pd/hour.

### Comparative Example B

The method of Example 4 was repeated, but using 20 ml propyne and 10 ml propadiene instead of 25 ml propyne, and heating to 60 °C. No reaction was observed. The autoclave was then heated to 80 °C. Again, no reaction was observed. The autoclave was finally heated to 100 °C. Reaction was then observed. The reaction time was 5 hours. The mean conversion rate was calculated to be only 200 g propyne and propadiene/g Pd/hour.

## Claims

1. A process for the preparation of an alkyl methacrylate, which comprises:
a) selectively removing propadiene from a C₃-mixture comprising a mixture of propyne and propadiene that has been obtained from an ethene cracker, a catalytic cracker or an LPG-dehydrogenation process, to afford a propyne feed in which the ratio of propyne to propadiene is ≧ 6, and
b) contacting the propyne feed with carbon monoxide and an alkanol in the presence of a carboxylation catalyst.

2. A process as claimed in claim 1, in which the C₃-mixture comprises a mixture of propyne and propadiene that has been obtained from an ethene cracker.

3. A process as claimed in claim 1 or claim 2, in which the C₃-mixture comprises a mixture of propyne and propadiene that has been obtained from an ethene cracker plant by taking at least part of the C₃-stream obtained from a depropaniser, introducing it directly into a propane/propene splitter and recovering the bottom effluent.

4. A process as claimed in any one of claims 1 to 3, in which the mixture of propyne and propadiene in the C₃-mixture has been concentrated by selective scrubbing with a solvent, whereby a solvent stream containing the C₃-mixture is obtained.

5. A process as claimed in claim 4, in which the solvent comprises dimethylformamide, N-methylpyrrolidone, methanol or a mixture thereof.

6. A process as claimed in any one of claims 1 to 5, in which propadiene is removed by extractive distillation.

7. A process as claimed in claim 4 or claim 5, in which propadiene is removed by stripping from the solvent stream.

8. A process as claimed in claim 6 or claim 7, in which propadiene which has been removed is contacted with an isomerisation catalyst in order to convert it into propyne, and the isomerisation product is recycled to step a) or step b).

9. A process as claimed in claim 8, in which the isomerisation catalyst comprises an alkali metal or alkali metal oxide on alumina.

10. A process as claimed in any one of claims 1 to 9, in which the propyne feed comprises at least 99 %(mass) of propyne.

11. A process as claimed in any one of claims 1 to 10, in which the ratio of propyne to propadiene in the propyne feed is ≧ 100.

12. A process as claimed in any one of claims 1 to 11, in which the carboxylation catalyst is a Group VIII metal catalyst.

13. A process as claimed in claim 12, in which the Group VIII metal catalyst is a palladium catalyst.

14. A process as claimed in any one of claims 1 to 13, in which the alkanol is methanol.

## Patentansprüche

1. Ein Verfahren zur Herstellung von Alkylmethacrylat, welches die folgenden Verfahrensstufen umfaßt:
a) selektives Entfernen von Propadien aus einer C₃-Mischung, umfassend eine Mischung von Propin und Propadien, die aus einem Ethencracker, einem katalytischen Cracker oder einem LPG-Dehydrierungsverfahren erhalten worden ist, um ein Propinausgangsmaterial zu erhalten, in welchem das Verhältnis von Propin zu Propadien ≧ 6 ist,
b) Kontaktieren des Propinausgangsmaterials mit Kohlenmonoxid und einem Alkanol in Gegenwart eines Carboxylierungskatalysators.

2. Ein Verfahren, wie in Anspruch 1 beansprucht, in welchem die C₃-Mischung eine Mischung aus Propin und Propadien umfaßt, die aus einer Ethencrackanlage gewonnen worden ist.

3. Ein Verfahren, wie in Anspruch 1 oder 2 beansprucht, in welchem die C₃-Mischung eine Mischung aus Propin und Propadien umfaßt, die aus einer Ethencrackanlage gewonnen worden ist, indem mindestens ein Teil des aus der Entpropanisierungsanlage erhaltenen C₃-Stroms entnommen und direkt in die Propan/Propen-Aufspaltanlage eingeführt, und der Bodenabstrom gewonnen wird.

4. Ein Verfahren, wie in einem der Ansprüche 1 bis 3 beansprucht, in welchem die Mischung aus Propin und Propadien in der C₃-Mischung durch selektives Auswaschen mit einem Lösungsmittel aufkonzentriert worden ist, wobei ein Lösungsmittelstrom erhalten wird, der die C₃-Mischung enthält.

5. Ein Verfahren, wie in Anspruch 4 beansprucht, in welchem das Lösungsmittel Dimethylformamid, N-Methylpyrrolidon, Methanol oder eine Mischung davon enthält.

6. Ein Verfahren, wie in einem der Ansprüche 1 bis 5 beansprucht, in welchem Propadien durch extraktive Destillation entfernt wird.

7. Ein Verfahren, wie in Anspruch 4 oder 5 beansprucht, in welchem Propadien durch Abstreifen aus dem Lösungsmittelstrom entfernt wird.

8. Ein Verfahren, wie in Anspruch 6 oder 7 beansprucht, in welchem Propadien, das entfernt worden ist, mit einem Isomerisationskatalysator kontaktiert wird, um es in Propin umzuwandeln, und das Isomerisationsprodukt zur Verfahrensstufe a) oder b) im Kreislauf rückgeführt wird.

9. Ein Verfahren, wie in Anspruch 8 beansprucht, in welchem der Isomerisationskatalysator ein Alkalimetall oder ein Alkalimetalloxid auf Aluminiumoxid enthält.

10. Ein Verfahren, wie in einem der Ansprüche 1 bis 9 beansprucht, in welchem die Propinzuspeisung mindestens 99%(Masse) Propin enthält.

11. Ein Verfahren, wie in einem der Ansprüche 1 bis 10 beansprucht, in welchem das Verhältnis von Propin zu Propadien im Propinausgangsmaterial ≧ 100 ist.

12. Ein Verfahren, wie in einem der Ansprüche 1 bis 11 beansprucht, in welchem der Carboxylierungskatalysator ein Katalysator eines Metalls der Gruppe VIII ist.

13. Ein Verfahren, wie in Anspruch 12 beansprucht, in welchem der Katalysator des Metalls der Gruppe VIII ein Palladiumkatalysator ist.

14. Ein Verfahren, wie in einem der Ansprüche 1 bis 13 beansprucht, in welchem das Alkanol Methanol ist.

## Revendications

1. Un procédé pour la préparation d'un méthacrylate d'alkyle, qui comprend :
a) l'élimination sélective du propadiène d'un mélange en C₃ comprenant un mélange de propyne et de propadiène qui a été obtenu à partir d'une unité de craquage d'éthène, d'une unité de craquage catalytique ou d'un procédé de déshydrogénation de GPL, pour donner une charge de propyne dans laquelle le rapport du propyne au propadiène est ≧ 6, et
b) la mise en contact de la charge de propyne avec de l'oxyde de carbone et un alcanol en présence d'un catalyseur de carboxylation.

2. Un procédé selon la revendication 1, dans lequel le mélange en C₃ comprend un mélange de propyne et de propadiène qui a été obtenu à partir d'une unité de craquage d'éthène.

3. Un procédé selon la revendication 1 ou la revendication 2, dans lequel le mélange en C₃ comprend un mélange de propyne et de propadiène qui a été obtenu à partir d'une unité de craquage d'éthène en prenant au moins une partie du courant de C₃ obtenu à partir d'un dépropaniseur, en l'introduisant directement dans une colonne de séparation propane/propène et en recueillant l'effluent du fond.

4. Un procédé selon l'une quelconque des revendications 1 à 3, dans lequel le mélange de propyne et de propadiène dans le mélange en C₃ a été concentré par lavage sélectif avec un solvant, de façon qu'on obtienne un courant de solvant contenant le mélange en C₃.

5. Un procédé selon la revendication 4, dans lequel le solvant comprend du diméthylformamide, de la N-méthylpyrrolidone, du méthanol ou un mélange de ces composés.

6. Un procédé selon l'une quelconque des revendications 1 à 5, dans lequel le propadiène est éliminé par distillation extractive.

7. Un procédé selon la revendication 4 ou 5, dans lequel le propadiène est éliminé par strippage du courant de solvant.

8. Un procédé selon la revendication 6 ou 7, dans lequel le butadiène qui a été séparé est mis en contact avec un catalyseur d'isomérisation de manière à être transformé en propyne, et le produit d'isomérisation est recyclé à l'étape a) ou à l'étape b).

9. Un procédé selon la revendication 8, dans lequel le catalyseur d'isomérisation comprend un métal alcalin ou un oxyde de métal alcalin sur de l'alumine.

10. Un procédé selon l'une quelconque des revendications 1 à 9, dans lequel la charge de propyne comprend au moins 99 % (en poids) de propyne.

11. Un procédé selon l'une quelconque des revendications 1 à 10, dans lequel le rapport du propyne au propadiène dans la charge de propyne est ≧ 100.

12. Un procédé selon l'une quelconque des revendications 1 à 11, dans lequel le catalyseur de carboxylation est un catalyseur à base d'un métal du groupe VIII.

13. Un procédé selon la revendication 12, dans lequel le catalyseur à base d'un métal du groupe VIII est un catalyseur au palladium.

14. Un procédé selon l'une quelconque des revendications 1 à 13, dans lequel l'alcanol est le méthanol.
